(19)

**Europäisches Patentamt**
**European Patent Office**
**Office européen des brevets**

(11) **EP 4 588 450 A1**

(12) **EUROPEAN PATENT APPLICATION**

(43) Date of publication:
**23.07.2025 Bulletin 2025/30**

(21) Application number: **25150199.5**

(22) Date of filing: **03.01.2025**

(51) International Patent Classification (IPC):
**A61B 18/14** (2006.01)   **A61B 18/00** (2006.01)

(52) Cooperative Patent Classification (CPC):
**A61B 18/1492;** A61B 2018/00577;
A61B 2018/00636; A61B 2018/00648;
A61B 2018/00702; A61B 2018/00773;
A61B 2018/00791; A61B 2018/00875

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **05.01.2024 US 202463617780 P
02.01.2025 US 202519008113**

(71) Applicant: **Biosense Webster (Israel) Ltd.
2066717 Yokneam (IL)**

(72) Inventors:
• **BAR-TAL, Meir
  2066717 Yokneam (IL)**

• **SIGAL, Alona
  2066717 Yokneam (IL)**
• **BERGER, Abraham
  2066717 Yokneam (IL)**
• **HAZAN, Ori Emanuel
  2066717 Yokneam (IL)**
• **OSADCHY, Daniel
  2066717 Yokneam (IL)**
• **MIZRAHI, Liron Shmuel
  2066717 Yokneam (IL)**

(74) Representative: **Carpmaels & Ransford LLP
One Southampton Row
London WC1B 5HA (GB)**

(54) **SYSTEM FOR CONTROLLING CARDIAC ABLATION USING BLOOD ELECTRICAL CONDUCTIVITY**

(57)    Methods and systems provide lesion size assessments to be ablated into tissue, such as cardiac tissue. The lesion sized assessments are based on parameters including blood conductivity, hot-spot-temperature, catheter tip temperature, and current data of the Radiofrequency (RF) ablation energy being used, such as power, duration, temperature, irrigation and contact force.

EP 4 588 450 A1

**Description**

**CROSS-REFERENCE TO RELATED APPLICATIONS**

This application claims the benefit of U.S. Provisional Patent Application 63/617,780, filed January 5, 2024, whose disclosure is incorporated herein by reference.

**TECHNICAL FIELD**

The present disclosure relates generally to tissue ablation procedures, and particularly to controlling the operation parameters of the ablation catheters which perform tissue ablation procedures.

**BACKGROUND**

Some medical procedures require the application of ablation signals to tissue of a patient's organ. For example, radiofrequency (RF) ablation signals may be applied to heart tissue at an ablation site for treating arrhythmia. Irreversible electroporation (IRE) / Pulsed Field Ablation (PFA) procedures utilize high-voltage pulses for catheter-based cardiac ablation to treat conditions such as symptomatic Atrial fibrillation by localized tissue necrosis.

**BRIEF DESCRIPTION OF THE DRAWINGS**

The present disclosure will be more fully understood from the following detailed description of the embodiments thereof, taken together with the drawings in which:

FIG. 1 is a schematic, pictorial illustration of an example system for performing ablation in accordance with the disclosed subject matter;
FIG. 2 is a schematic diagram of a feedback loop used with the system of FIG. 1, in accordance with the disclosed subject matter;
FIGs. 3A and 3B, collectively referred to hereinafter as FIG. 3, are a flow diagram of an example lesion assessment algorithm, in accordance with the disclosed subject matter; and
FIG. 4 is a flow diagram of an example process to decide whether a patient is to be excluded from a procedure, in accordance with the disclosed subject matter.

**DETAILED DESCRIPTION OF EXAMPLES**

OVERVIEW

Ablation catheters are used to create ablated lesions of dead or necrosed tissue for various procedures. The term "ablation", as used herein, refers to a radiofrequency (RF) ablation procedure or to an irreversible electroporation (IRE) procedure, sometimes also referred to as Pulsed Field Ablation (PFA). These procedures are intended to apply one or more high-voltage unipolar or bipolar electrical pulses to one or more electrodes in contact with tissue to be ablated, so as to form a lesion in the target location (also referred to herein as an ablation site) in heart, and thereby to treat arrhythmia in the heart.
For example, in RF ablation procedures, parameters applied to the ablation catheter and the generator include power, duration, temperature, irrigation and contact force. These parameters are defined to obtain a desired lesion, for example, of a desired depth into the tissue and lateral size (e.g., diameter).
During ablation some of the energy gets absorbed by the blood pool. For example, with RF ablation in the heart muscle, most of the current and the electrical power generated by the generator, approximately 75-80% flows to the blood, and is absorbed by the blood, with only a small amount, 20-25% entering the tissue. This absorption is believed to be caused by the surface area of the tip of the ablation catheter touching the blood being much larger than the effective surface area that contacts the tissue, and the electrical conductivity of the blood is higher than that of the tissue.
When performing ablation procedures, the depth and lateral size of lesion varies by patient. To accommodate the differences between patients, lesion parameters are adjusted based on the individual patient. However, it is difficult to predict the parameters of a lesion per patient. Further difficulties in lesion size prediction are due to the range of blood conductivity in humans being very wide (e.g., blood impedance experienced by the electrode varies between 100 to 150 Ohms), significantly affecting the amount of energy that flows to the tissue during RF ablation.
For example, generally, the higher the blood conductivity, the more RF energy (ablation current) emitted from the ablation catheter flows to the blood (where it is absorbed, rather than flowing to the tissue to be ablated.

To accurately calculate the desired lesion size for the tissue of an individual patient, the orientation of the ablation catheter needs to be analyzed for the quality of the contact between the ablation catheter tip and the tissue, and the patient's blood electrical conductivity should be known. Blood conductivity can be determined from a blood sample prior to the ablation procedure. Based on these two factors, the ablation catheter and its generator can be controlled to produce a lesion of the desired depth and lateral size, such that the lesion is uniform among patients.

The lesion size assessment is performed by an algorithm, which uses parameters including blood conductivity, catheter tip temperature, and current data of the RF ablation energy being used, such as power, duration, temperature, irrigation, and contact force. The use of actual, measured values for blood conductivity, along with the measured catheter tip temperature, impedance, and contact force, was found to achieve a substantially more accurate lesion assessment.

SYSTEM DESCRIPTION

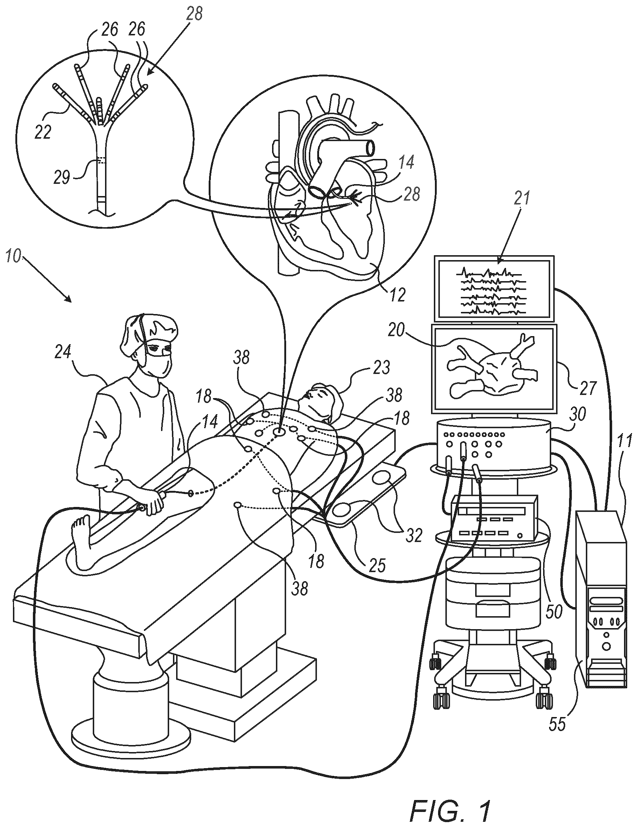

Reference is made to FIG. 1, which schematically illustrates an example catheter-based electrophysiology mapping and ablation system 10 that includes multiple catheters, which are percutaneously inserted by a physician 24 through the patient's vascular system into a chamber or vascular structure of a heart 12. Typically, a delivery sheath catheter is inserted into the left or right atrium near a desired location in heart 12. Thereafter, one or more catheters may be inserted into the delivery sheath catheter so as to arrive at the desired location in heart 12. The plurality of catheters may include catheters dedicated for sensing Intracardiac Electrogram (IEGM) signals, catheters dedicated for ablating and/or catheters dedicated for both sensing and ablating.

An example catheter 14 that is configured for sensing IEGM is illustrated herein. The physician 24 may place a distal tip 28 of catheter 14 in contact with the heart wall for sensing a target site in heart 12. For ablation, the physician 24 may similarly place a distal end of an ablation catheter in contact with a target site for ablating tissue. For example, the ablation catheter may comprise a SMARTTOUCH® or VARIPULSE® catheter produced by Biosense Webster Inc. (Irvine, California).

The catheter 14 is, for example, a catheter that includes one or more electrodes 26 optionally distributed over a plurality of splines 22 at distal tip 28 and configured to sense the IEGM signals. The electrodes 26 can be used to make impedance measurements, from which blood conductivity may be derived, coordinated by the workstation 55 and processor. For example, impedance measurement estimation may use impedance measured at least between any two catheter electrodes 26.

The catheter 14 may additionally include one or more position sensors, shown for example, by the position sensor 29, which is embedded in or near the distal tip 28. The position sensor(s) 29 functions in tracking the position and orientation of the distal tip 28 of the catheter 14. Optionally, position sensor 29 is a magnetic based position sensor including one to three magnetic coils for sensing three-dimensional (3D) position and orientation of the catheter 14 and the catheter tip 28.

Magnetic position sensor 29 may be operated together with a location pad 25 including a plurality of magnetic coils 32 configured to generate magnetic fields in a predefined working volume. The real-time position of the distal tip 28 of the catheter 14 may be tracked based on magnetic fields generated with the location pad 25 and sensed by the magnetic based position sensor 29. Details of the magnetic based position sensing technology used by the catheter 14 and magnetic position sensor(s) 29, are described, for example, in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

The system 10 includes one or more electrode patches 38, which are positioned for skin contact on the patient 23 to establish location reference for location pad 25 as well as impedance-based tracking of the electrodes 26. For impedance-based tracking, electrical current is directed to the electrodes 26 and sensed at electrode skin patches 38 so that the location of each electrode can be triangulated via the electrode patches 38. Details of the impedance-based location tracking technology are described, for example, in US Patent Nos. 7,536,218; 7,756,576; 7,848,787; 7,869,865; and 8,456,182.

A recorder 11 records and displays electrograms 21 captured with body surface ECG electrodes 18 and intracardiac electrograms (IEGM) captured with electrodes 26 of the catheter 14. The recorder 11 may include pacing capability for pacing the heart rhythm and/or may be electrically connected to a standalone pacer.

The system 10 may include an ablation energy generator (RF Generator) 50 that is adapted to conduct ablative energy to one or more electrodes at a distal tip of a catheter configured for ablating. The ablation energy may be delivered by catheter 14 or by an additional ablation catheter (not shown). Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE)/ Pulsed Field Ablation (PFA), or combinations thereof. The energy produced, for example, RF energy, is based on various ablation parameters, including, for example, catheter tip 28 contact quality and the blood conductivity ($\sigma$) of the patient 23.

A patient interface unit (PIU) 30 is an interface configured to establish electrical communication between catheters, other electrophysiological equipment, power supplies, and a workstation 55, for controlling the operation of system 10. The electrophysiological equipment of system 10 may include, for example, multiple catheters, location pad 25, body surface ECG electrodes 18, electrode patches 38, ablation energy generator 50, and the recorder 11. Optionally, the PIU 30 additionally includes processing capability for implementing real-time computations of the location of the catheters and for performing ECG calculations.

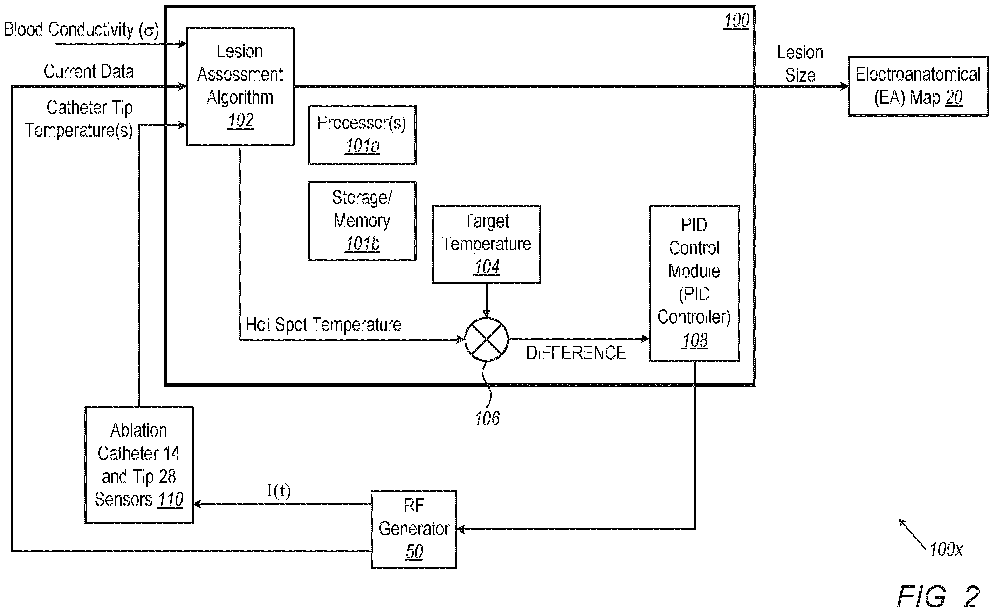

The workstation 55 includes memory, a processor unit 101a (one or more processors including, for example a central processing unit (CPU)) with memory or storage 101b (FIG. 2)) and appropriate operating software stored therein. The processor unit 101a and memory 101b also includes and/or provides, for example, and user interface capability, through which users can input various parameters, either manually or automatically through the PIU 30, such as blood conductivity, ablation catheter tip 28 temperatures (also referred to as tip temperatures), hot spot temperatures (hot spot temperatures are the area of maximum or hottest temperature inside and ablated lesion), target temperatures (t), where t < 140 Celsius, and the like. The workstation 55 also includes a Proportional-Integral-Derivative (PID) control module 108 (FIG. 2) and via its processor(s) also runs software for a lesion assessment algorithm 102 (FIG. 2).

The workstation 55, for example, also provides multiple functions, such as one or more of: (1) modeling the endocardial anatomy in three-dimensions (3D) and rendering the model or electroanatomical (EA) map 20 for display on a display device 27, (2) displaying on the display device 27 activation sequences (or other data) compiled from recorded electrograms 21 in representative visual indicia or imagery superimposed on the rendered electroanatomical map 20, (3) displaying real-time location and orientation of multiple catheters within the heart chamber, (4) displaying on display device 27 sites of interest such as places where ablation energy has been applied, and (5) providing optimal ablation parameters for the ablation including those for catheter tip contact quality and the patient's blood conductivity. One commercial product embodying elements of system 10 is available as the CARTO™ 3 System, available from Biosense Webster, Inc., 31A Technology Drive, Irvine, CA 92618.

The workstation 55 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

ABLATION CONTROL SYSTEM AND LESION ASSESSMENT MODEL

FIG. 2 shows schematically an ablation control system 100 as part of a feedback control loop 100x. The ablation control system 100 is a subsystem within the main system 10, and is, for example, within the workstation 55. System 100 includes components, for example, processor(s) 101a and associated storage/memory 101b, which run the lesion assessment algorithm 102. System 100 also includes a target temperature module 104, a mixer 106, and a Proportional Integral Derivative (PID) control module (PID controller 108). The system 100 components 101a, 101b, 102, 104, 106, 108 are, for example, in direct or indirect electronic and/or data communication with each other.

The processor(s) 101a include one or more processors, including microprocessors, for controlling operation of the lesion assessment algorithm 102, the target temperature module 104, the mixer 106 and/or the PID control module 108, as well as performing the functions and operations detailed herein. Typically, the processor(s) 101a comprise a general-purpose computer, which is programmed in software to carry out the functions described herein. Software may be downloaded to the computer in electronic form, over a network, for example, or it may, alternatively or additionally, be provided and/or stored on non-transitory tangible media, such as magnetic, optical, or electronic memory. The processors are, for example, conventional processors, such as those used in servers, computers, and other computerized devices, including hardware processors. For example, the processors may include x86 Processors from AMD (Advanced Micro Devices®) and Intel®, Xenon® and Pentium® processors from Intel, as well as any combinations thereof.

The storage/memory 101b includes any conventional storage media. The storage/memory 204 stores machine executable instructions for execution by the processor(s) 101a, to perform the disclosed processes.

The lesion assessment algorithm 102 receives inputs of blood conductivity (6) for example, tip temperatures of the ablation catheter 14 as detected by sensors, and current data of ablation energies, from the RF ablation generator 50. From these inputs, the lesion assessment algorithm 102 performs calculations to provide ablation energy to be output from the RF generator 50 to the ablation catheter, e.g., catheter 14, to ablate the lesion of the calculated or otherwise assessed lesion size. Details of the lesion assessment algorithm 102 are presented in an Appendix, which is an integral part of the present disclosure.

Blood conductivity ($\sigma$), in units of Siemens/meter (S/M), from blood of a blood pool of the patient 23, may be obtained by one or more of the following methods:

1. Direct Measurement - The direct measurements of blood conductivity are performed with probes, such as an Eutech™ ECTestr11 Dual Range Conductivity Tester, from Thermo Scientific, measuring a blood sample. The blood sample is, for example, an at least 5 cc sample of venous blood from the femoral vein, the sample measured by the probe.

2. Red Blood Cell (RBC) Count - A red blood cell count is taken from a blood sample from the patient, for example, the blood sample detailed above for the "Direct Measurement". A correlation, as detailed, for example, in FIG. 1 of Texter, Jr., et al. "The Electrical Conductivity of Blood - II. Relation and Cell Count", in Blood, Volume 5, Issue 11, November 1950, Pages 1036-1048, this document incorporated by reference in its entirety herein. The correlation converts the red blood cell count into the aforementioned electrical conductivity value in S/m.

3. Impedance Measurements Between catheter 14 ring Electrodes 26 - This method involves measuring blood impedance between at least two catheter electrodes 26 and, in some cases, for example, converting the obtained values for the impedance measurements to electrical conductivity using a graph or calibration table, as detailed, for example, in commonly owned US Patent Nos. 10,398,348 and 11,596,324, the disclosures of which are incorporated by reference in their entireties herein.

The catheter tip temperature is measured by one or more temperature sensors located at the tip of the catheter. The temperature measurement is updated continually, for example at a rate of 20 Hz.

For the purposes of algorithm 102, the catheter penetration depth is initially estimated based on contact force and/or impedance measurements between the ablating electrode and the tissue surface of the patient.

The lesion assessment algorithm 102, also known as a lesion size assessment algorithm, outputs a lesion size, which is projected on the electroanatomical (EA) map 20, for viewing by the physician 24 and/or control room operator. Also output are hotspot temperatures, which are compared to target temperatures, and the difference between them (computed, for example by the mixer 106), is input TO the PID control module 108.

The hot spot temperature is calculated, for example, by a thermal model, for example as described in the Appendix. The hot spot temperature, for example, is the maximum temperature inside the ablated area of tissue, as determined, for example, based on measurements made by temperature sensors 110 in the ablation catheter tip.

The target temperature is determined by the processor(s) 101a based on temperatures sufficient for ablation which will not result in steam pop, for example, as disclosed in commonly owned U.S. Patent No. 9,241,756 the disclosure of which is incorporated by reference herein, and is, for example, typically less than 140 degrees Celsius. Alternatively, target temperatures may be stored in a database or in the PID module 108, for example, as a look up table (LUT) or the like.

The PID controller 108, which receives the "error" value, and based on this value, functions to control the current supply of RF energy necessary to ablate the ablation spot of the sized determined by the lesion assessment algorithm 102. For example, the PID controller 108, calculates output signals, to control the RF ablation energy and the pulses thereof, generated by the RF generator 50. The calculated pulses are used by the ablation catheter 14 to ablate a lesion of the desired dimensions, in accordance with the lesion size calculation, as determined (e.g., calculated) by algorithm 102.

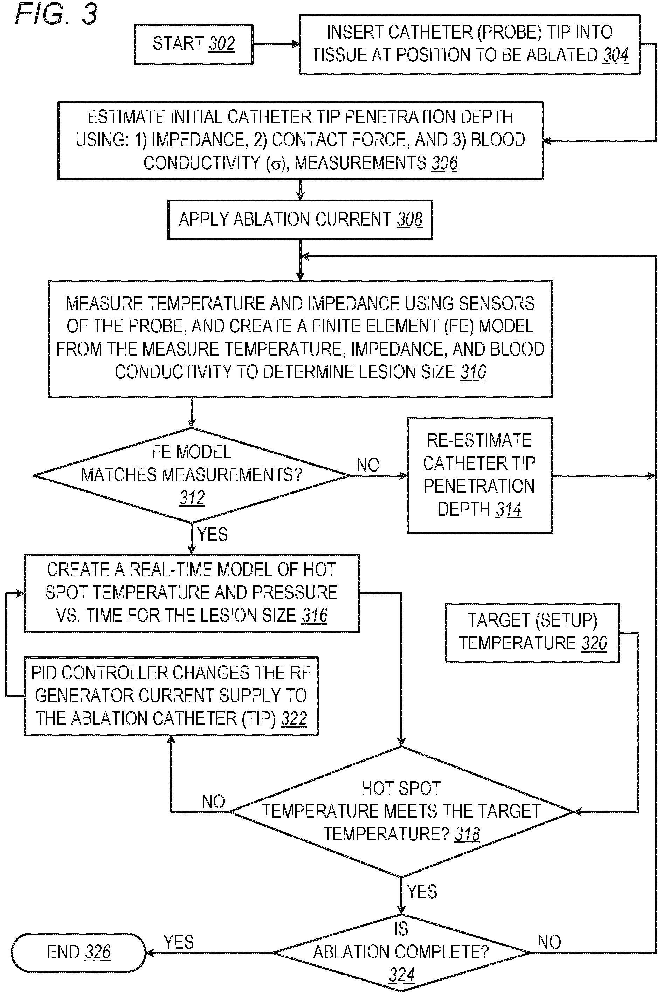

Attention is now directed to FIG. 3, which shows a flow diagram detailing computer-implemented processes, as performed, for example, by the lesion assessment algorithm 102. Reference is also made to elements shown in FIGs. 1 and 2. The process and sub-processes of FIG. 3 are computerized processes performed by the system 10 and subsystem 100. The aforementioned processes and sub-processes are, for example, performed continuously, automatically and, for example, in real time.

The lesion assessment begins at a START block 302. The processor has been programmed with physical parameters and material properties, such as thermal and electrical properties of the material of tip 28 of ablation catheter 14. The processor is also programmed to cause sampling at regular intervals, for example, at a rate of 20 times per second, from six different probes (e.g., sensors 29) at tip 28 of catheter 14.

Moving to block 304, ablation catheter 14 positioning begins with the physician 24 inserting the ablation catheter 14 into the tissue to be ablated. The physician 24 may use any suitable method to select an ablation site within the tissue. For example, in heart ablation, the physician 24 may select to ablate sites of arrhythmias as known in the art.

Following probe positioning, the process moves to block 306, where the processor(s) 101a estimates the initial penetration depth of the tip 28 of the catheter 14 into the tissue, for example, using one or more of a) impedance, contact force and/or temperature, measurements, and b) blood conductivity measurements. For example, the impedance is determined by impedance measurements from electrodes 26, e.g., between two or more on the catheter 14. For example, a Tissue Proximity Indication (TPI) algorithm may be used to measure the impedance between electrodes 26 to estimate catheter 14/catheter tip 28 penetration depth, the TPI algorithm as detailed in US Patent No. 10,398,348.

The contact force is determined by measurements on force gauges (not shown) near the catheter tip 28 that measure the contact force on the tissue, for example, at a resolution of 1 gram. Temperature measurements may be made, for example, by penetration of the tip 28 into the tissue (equal to tip surface coverage by the tissue), for example, as disclosed in J. Kosuth, et al., "Chamber-Specific Radiofrequency Lesion Dimension Estimation Using Novel Catheter-Based Tissue Interface Temperature Sensing", in JACC: Clinical Electrophysiology, Vol. 3, No. 10, pages 1092-1102 (2017), the disclosure of which is incorporated by reference herein.

The process moves to block 308, where the RF generator 50 is activated to apply ablation current to the tissue. The RF power level generated by RF generator 50 is controlled by the current level applied to the generator 50. Energy created by RF generator 50 is delivered via the ablation catheter 14 to the tissue at the site of ablation. Typically, as RF energy is applied to the tissue, the ablation catheter 14 performs temperature, impedance and force measurements that are delivered to the processor(s) 101a.

Moving to block 310, the processor (s) 101a uses the estimated tip 28 depth (from block 306) and the blood conductivity ($\sigma$), as well as present actual temperature and impedance measurements made at the catheter tip 28, to create a finite element (FE) model. The FE model comprises calculated temperature and impedance values at multiple sub-regions of a 3D region that is referred to as a temperature domain. The temperature domain typically comprises a region of the tissue, e.g., region and hot spot, the tip 28 of the catheter 14 and sensors, and a region in close vicinity of the tissue wherein liquids such as blood and irrigation saline may be present. Note that sensors at the tip 28 of the ablation catheter 14 reside in some of the sub-regions of the FE model. An example FE model is that disclosed in, J. Kosuth, et al., "Chamber-Specific Radiofrequency Lesion Dimension Estimation Using Novel Catheter-Based Tissue Interface Temperature Sensing", in JACC: Clinical Electrophysiology, as detailed above. Implementation of the FE model in the present embodiment is described further in the Appendix.

The process moves to block 312, where the processor (s) 101a compares the calculated temperature and impedance values of the FE model to actual measurements of the probe sensors. In case there is no match at block 312, the processor re-estimates the tip penetration depth at a re-estimation step 314 and loops back to step 310, from where the process resumes. The loop of re-estimation of the penetration depth at block 314, by repeating the process of block 306, coupled with and adjusting the FE model (of block 310), continues until processor 101a finds a match at block 312.

If there is a match, at block 312, the process proceeds to block 316, where a real-time model of the predicted hotspot temperature and pressure evolution as a function of time is created for the given blood conductivity and lesion size. The real-time model is based at least on the tip (probe) penetration depth (which is a "free parameter" of the model) estimated at blocks 306 or 314, and on the ablation current used to activate RF generator 50. The model typically comprises a predicted evolution curve of the temperature and/or pressure at the hot spot. Alternatively, the model may comprise a table of temperature/pressure and time points. Further alternatively, the model may comprise any other suitable form, such as a mathematical formula.

Based on the model and on predefined temperature/pressure thresholds, the process moves to block 318, where it is determined whether a now-determined hot spot temperature meets the target temperature, at block 320. The target temperature is the desired temperature for an ablation spot, which is high enough to create tissue necrosis while avoiding steam pops, and is, for example, obtained from a look-up table or the like.

Returning to block 318, if the hot spot temperature does not meet the target temperature (as programmed into the system 100), the process moves to block 322. At block 322 the PID controller (PID control module 108) changes the RF generator 50 current supply to the tip of the catheter 28, in accordance with the difference between the hot spot temperature and the target temperature. The process then moves to block 316, from where it resumes.

Alternatively, at block 318, if the hot spot temperature meets the target temperature, the process moves to block 324. At block 324 the processor determines whether the ablation is complete. For example, to determine whether ablations are complete, physician 24 may terminate the ablation if the amount of energy (e.g., the product of the RF power and time duration) applied to the tissue exceeds a predefined threshold.

Alternatively, or additionally, the physician 24 may decide on ablation termination by consulting any suitable information presented on display 27 during the ablation process. Further alternatively or additionally, the processor may automatically decide on ablation termination using, for example, methods described in U.S. Patent No. 8,900,225, the disclosure of which is incorporated in its entirety herein by reference.

At block 324, when the ablation is complete, the process (method) of the lesion assessment algorithm 102 terminates at a termination step 326.

Otherwise, at block 324, should the ablation not be complete, the processor loops back to step 310 to re-adjust the FE model in case deviations to the model have occurred. Factors that may cause FE model deviations include, for example, tissue-probe relative motion created by heart beats and respiration operations of patient 23. Note that adjusting the FE model at step 310 may be followed by respective updates to the real-time model at block 316, to the predicted occurrence time of the steam pop event at block 320.

The process may be repeated for as long as desired.

Alternatively, or additionally, the physician 24 may adjust the position or penetration depth of the catheter 14 into the tissue, or even temporarily remove the catheter from being in contact with the tissue. The physician 24 may apply increased force upon the catheter 14 to insert it deeply into the tissue if the prediction model indicates a slow rising temperature/pressure curve. In case, however, the model indicates an imminent occurrence of a steam pop event, the physician 24 may partially or completely remove the catheter 14 from the tissue. Further alternatively or additionally, the physician 24 may control irrigation rate, and/or any other suitable means that may affect temperature/pressure evolution rate, responsively to the model indication. The physician 24 may also respond with various actions in parallel to reduce the risk for a stem pop event.

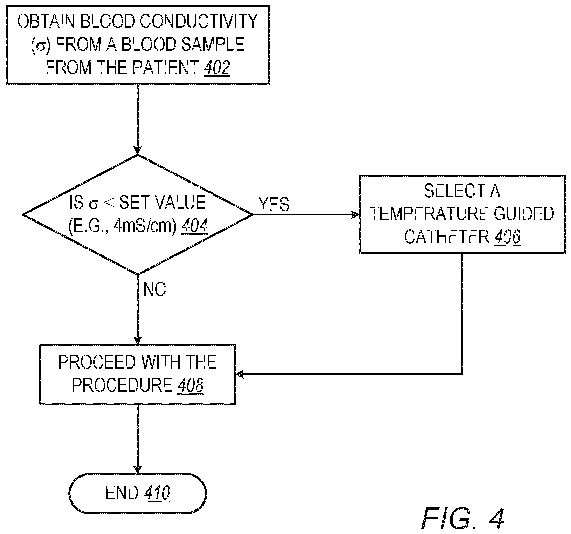

FIG. 4 illustrates a flow diagram of a process to determine whether a patient is a suitable candidate for ablation based on blood conductivity. For example, when the electrical conductivity of blood is below a certain value, approximately 4 microSemens/centimeter(mS/cm), more of the ablation current flows to the tissue, as compared to normal blood conductivity of approximately 6.5 mS/cm. When this large current amount reaches the tissue, the tissue heats up faster resulting in a larger lesion which come with risk factors, such as steam pop, charring, and/or esophageal overheating.

The process begins at block 402, where blood conductivity is obtained by one of the processes detailed above. The process moves to block 404, where it is determined whether the blood conductivity measurement is less than a threshold, e.g., 4 mS/cm.

If yes at block 404, the process moves to block 406, where a temperature guided (e.g., controlled) catheter is selected for a procedure. Such catheter's temperature sensor continuously monitors the temperature at the tip, which is where the ablation happens. This real-time feedback allows adjusting the procedure fast enough, depending on how the patient's tissue reacts, to avoid damage to healthy tissue. The process then moves to block 408, where the ablation procedure proceeds.

Alternately, if no at block 404, the process moves directly to block 408, where the ablation procedure proceeds.

From block 408, the process moves to block 410, where the ablation procedure ends.

Although the examples described herein mainly address ablation of cardiac tissue, other tissues may also be ablated by the apparatus and methods disclosed herein.

The implementation of the method and/or system of examples of the disclosure can involve performing or completing selected tasks manually, automatically, or a combination thereof. Moreover, according to actual instrumentation and equipment of examples of the method and/or system of the disclosure, several selected tasks could be implemented by hardware, by software or by firmware or by a combination thereof using an operating system or a cloud-based platform.

For example, hardware for performing selected tasks according to examples of the disclosure could be implemented as a chip or a circuit. As software, selected tasks according to examples of the disclosure could be implemented as a plurality of software instructions being executed by a computer using any suitable operating system. In an exemplary example of the disclosure, one or more tasks according to exemplary examples of method and/or system as described herein are performed by a data processor, such as a computing platform for executing a plurality of instructions. Optionally, the data processor includes a volatile memory for storing instructions and/or data and/or a non-volatile storage, for example, non-transitory storage media such as a magnetic hard-disk and/or removable media, for storing instructions and/or data. Optionally, a network connection is provided as well. A display and/or a user input device such as a keyboard or mouse are optionally provided as well.

For example, any combination of one or more non-transitory computer readable (storage) medium(s) may be utilized in accordance with the above-listed examples of the present disclosure. The non-transitory computer readable (storage) medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store, a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

As will be understood with reference to the paragraphs and the referenced drawings, provided above, various examples of computer-implemented methods are provided herein, some of which can be performed by various examples of apparatuses and systems described herein and some of which can be performed according to instructions stored in non-transitory computer-readable storage media described herein. Still, some examples of computer-implemented methods provided herein can be performed by other apparatuses or systems and can be performed according to instructions stored in computer-readable storage media other than that described herein, as will become apparent to those having skill in the art with reference to the examples described herein. Any reference to systems and computer-readable storage media with respect to the following computer-implemented methods is provided for explanatory purposes, and is not intended to limit any of such systems and any of such non-transitory computer-readable storage media with regard to examples of computer-implemented methods described above. Likewise, any reference to the following computer-implemented methods with respect to systems and computer-readable storage media is provided for explanatory purposes, and is not intended to limit any of such computer-implemented methods disclosed herein.

The flowchart and block diagrams in the Figures illustrate the architecture, functionality, and operation of possible implementations of systems, methods and computer program products according to various examples of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, block diagrams and/or flowchart illustration, and combinations of blocks in the block diagrams and/or flowchart illustration, can be implemented by special purpose hardware-based systems that perform the specified functions or acts, or combinations of special purpose hardware and computer instructions. The descriptions of the various examples of the present disclosure have been presented for purposes of illustration, but are not intended to be exhaustive or limited to the examples disclosed. Many modifications and variations will be apparent to those of ordinary skill in the art without departing from the scope and spirit of the described examples.

LESION ASSESSMENT ALGORITHM

The disclosed lesion assessment algorithm (102) is used to evaluate lesion parameters using the temperature(s) measured by the therapeutic catheter. The lesion parameters are lesion size (width and depth) and the maximum temperature developing in the tissue (Hot Spot).

The algorithm described in the following sections runs in real time, but is based on data compiled offline, look-up tables (LUT) and material properties collected beforehand. This contributed both to the algorithm's relative simplicity and to its speed, without detracting noticeably from its accuracy.

Variations in measurement due to heartbeat and even respiration have little effect on the lesion formation, because the thermal timescale (the time it takes a temperature front to propagate in the spatial scale of the problem, or for the temperature field to change significantly) is of the order of several seconds.

Heartbeat and respiration motion cause movement of the catheter relative to the tissue, which in turn causes changes in the measured temperature as the sensors move relative to the lesion.

Conversely, the heat generation in the tissue can remain steady, helped by the large heat capacitance of the tissue.

The tissue heat capacity thus acts as a low-pass filter, averaging the fluctuations.

**BASIC OPERATING PRINCIPLES**

Offline (Generate a Look up table (LUT) model):

- Perform all the simulations for the relevant ablation setup (current, catheter penetration/depth, impedance, ablation time, blood conductivity, etc.)

Run-time:

1. Start an ablation and measure the data from catheter's sensors (Temperature, impedance).
2. The measured values' behavior is unique and similar to the values produced by a particular simulation (particular in the sense that it was produced using a specific catheter depth, current, material properties etc.)
The data for this simulation can be retrieved from the relevant look-up table.
3. The simulation output look-up tables include parameters which are not measured directly, including the extent and form of the temperature field in the tissue and the values of measurements we would have obtained if reality was identical to the simulation. By continuously comparing the measured and the simulated temperature, the optimal simulation scenario database is selected from which the **lesion size** and **max temperature** (hot spot) are extracted.

**SIMULATIONS AND MODEL GENERATION**

In order to perform the simulations needed to produce the data used by the algorithm the following parameters are needed:

1. The **catheter tip structure** including the insert and the temperatures sensors.
2. Range of **power/current** applied during ablation.
3. Range of catheter **penetration depths/angles** specifying the contact geometry.
4. **Material properties** (e.g. thermal/electric conductivity, temperature ranges, irrigation flow rates, etc.)
5. Ablation **site properties** (RA, RV, geometry of the tissue and beyond such as the existence of a fat layer/lung/pericardium) .
6. Range of **blood conductivity.**

Multiple parameter combinations from the set above are used to produce the database that constitutes the algorithm input (i.e., LUT).

All the models are run assuming that the ablation duration is up to 125 sec., with currents in the range covering the range used in the electrophysiology ablation practice.

Each simulation generates a time-dependent temperature field. These results can be parsed for the data relevant to the algorithm, such as lesion width/depth (lesion border defined as the 57C isotherm, and maximum tissue temperature (hot spot)).

After completion of the simulations covering all the parameter combinations and variations, the following results are extracted into the algorithm input database:

- Lesion Depth
- Lesion Width
- Measured Temperature
- Temperature at the hottest tissue spot.

All the parameters are organized as tables of the form:

$$Value(time = t, penetration = D, current = I, Conductivity = C)$$

so that each value corresponds to a particular time, catheter penetration depth, ablation current, and blood conductivity.

Each parameter is described as a function of (t,D,I,C) by using linear interpolation of the values within the (t,D,I,C) set. This provides a continuous function to be used as algorithm predicting the lesion progress.

**DATA PRE-PROCESSING**

MAXIMUM MEASURED TEMPERATURE:

Of all the temperature measurements (for example, six measurements in the QDOT catheter), only the maximum value is transferred to the algorithm. The implicit assumption is that the sensor with the highest measured temperature is the one closest to the lesion and thus best suited to evaluate it.

INSTANTANEOUS LOSS OF CONTACT:

As long as the catheter tip is in contact with the tissue and the ablation current heats the tissue, local decrease in tissue temperature cannot be fast.

Therefore, when the measured temperature exhibits a rapid decrease, we can assume that the temperature decreases measured are due to instantaneous loss of contact by the measuring sensor, and not true decrease of tissue temperature. This intermittent contact represents the tissue temperature during its 'high' points, and only these points would represent the tissue temperature correctly.

EXAMPLES

Example 1

A method for performing ablation of tissue of a patient with an ablation catheter (14), the method comprising obtaining (402) a blood conductivity value for blood of a blood pool associated with the ablation catheter and the tissue to be ablated. Ablation energy needed to ablate the tissue is calculated (316) based on ablation parameters including the blood conductivity value. A size of a lesion to be created by the ablation energy is assessed (310) based on the calculated ablation energy.

Example 2

The method according to example 1, wherein calculating (316) the ablation energy further comprises applying a hot spot temperature of an ablated lesion to the calculation.

Example 3

The method according to example 1, wherein assessing (310) the lesion size further comprises estimating (306) an initial penetration depth of a tip of the catheter into the tissue, and, using the estimated initial penetration depth and the blood conductivity value, as well as a present actual temperature and impedance measurements made at the tip, to create a finite element (FE) model of a three-dimensional (3D) temperature distribution in the tissue and estimating (316) the size of the lesion from the temperature distribution.

Example 4

The method according to example 3, and comprising adjusting (322) the ablation energy based on the size of the lesion.

Example 5

The method according to example 1, and comprising selecting (406, 408) a catheter type based on the obtained blood conductivity value.

Example 6

The method according to example 1, wherein obtaining (402) the blood conductivity value comprises determining the blood conductivity value from a blood sample of the patient prior to the ablation.

Example 7

The method according to example 1, wherein assessing (310) the size of the lesion comprises using premade look-up tables of entries for lesion depth, lesion width, measured temperature, and temperature at a hottest tissue spot.

Example 8

The method according to example 1, wherein assessing (310) the size of the lesion comprises assessing a depth and a lateral size of the lesion.

Example 9

A system (10) for performing ablation of tissue of a patient with an ablation catheter (14), the system includes an interface (30) and a processor (101a). The interface is configured to obtain (402) a blood conductivity value for blood of a blood pool associated with the ablation catheter and the tissue to be ablated. The processor (101a) is configured to calculate (316) ablation energy needed to ablate the tissue based on ablation parameters, including the blood conductivity value, and, based on the calculated ablation energy, assess (310) a size of a lesion to be created by the ablation energy.
As used herein, the singular form "a", "an" and "the" include plural references unless the context clearly dictates otherwise.
It is appreciated that certain features of the disclosure, which are, for clarity, described in the context of separate examples, may also be provided in combination in a single example. Conversely, various features of the disclosure, which are, for brevity, described in the context of a single example, may also be provided separately or in any suitable sub-combination or as suitable in any other described example of the disclosure. Certain features described in the context of various examples are not to be considered essential features of those examples, unless the example is inoperative without those elements.

**Claims**

1.  A method for performing ablation of tissue of a patient with an ablation catheter (14), the method comprising:

obtaining (402) a blood conductivity value for blood of a blood pool associated with the ablation catheter and the tissue to be ablated;

calculating (316) ablation energy needed to ablate the tissue based on ablation parameters including the blood conductivity value; and

based on the calculated ablation energy, assessing (310) a size of a lesion to be created by the ablation energy.

2. The method according to claim 1, wherein calculating (316) the ablation energy further comprises applying a hot spot temperature of an ablated lesion to the calculation.

3. The method according to any of claims 1 and 2, wherein assessing (310) the lesion size further comprises estimating (306) an initial penetration depth of a tip of the catheter into the tissue, and, using the estimated initial penetration depth and the blood conductivity value, as well as a present actual temperature and impedance measurements made at the tip, to create a finite element (FE) model of a three-dimensional (3D) temperature distribution in the tissue and estimating (316) the size of the lesion from the temperature distribution.

4. The method according to any of claims 1 through 3, and comprising adjusting (322) the ablation energy based on the size of the lesion.

5. The method according to any of claims 1 through 4, and comprising selecting (406, 408) a catheter type based on the obtained blood conductivity value.

6. The method according to any of claims 1 through 5, wherein obtaining (402) the blood conductivity value comprises determining the blood conductivity value from a blood sample of the patient prior to the ablation.

7. The method according to any of claims any of claims 1 through 6, wherein assessing (310) the size of the lesion comprises using premade look-up tables of entries for lesion depth, lesion width, measured temperature, and temperature at a hottest tissue spot and/or assessing a depth and a lateral size of the lesion.

8. A system (10) for performing ablation of tissue of a patient with an ablation catheter (14), the system comprising:

an interface (30) configured to obtain (402) a blood conductivity value for blood of a blood pool associated with the ablation catheter and the tissue to be ablated; and

a processor (101a), which is configured to:

calculate (316) ablation energy needed to ablate the tissue based on ablation parameters including the blood conductivity value; and

based on the calculated ablation energy, assess (310) a size of a lesion to be created by the ablation energy.

9. The system according to claim 8, wherein the processor (101a) is configured to calculate (316) the ablation energy by applying a hot spot temperature of an ablated lesion to the calculation.

10. The system according to any of claims 8 and 9, wherein the processor (101a) is configured to assess (310) the lesion size by estimating (306) an initial penetration depth of a tip of the catheter into the tissue, and, using the estimated initial penetration depth and the blood conductivity value, as well as a present actual temperature and impedance measurements made at the tip, to create a finite element (FE) model of a three-dimensional (3D) temperature distribution in the tissue and estimating (316) the size of the lesion from the temperature distribution.

11. The system according to any of claims 8 through 10, wherein the processor (101a) is configured to adjust (322) the ablation energy based on the size of the lesion.

12. The system according to any of claims 8 through 11, wherein the processor (101a) is configured to select (406, 408) a catheter type based on the obtained blood conductivity value.

13. The system according to any of claims 8 to 12, wherein the interface (30) is configured to obtain (402) the blood conductivity value by determining the blood conductivity value from a blood sample of the patient prior to the ablation.

14. The system according to any of claims 8 to 13, wherein the processor (101a) is configured to assess (310) the size of the lesion by using premade look-up tables of entries for lesion depth, lesion width, measured temperature, and temperature at a hottest tissue spot.

15. The system according to any of claims 8 to 14, wherein the processor (101a) is configured to assess (310) the size of the lesion by assessing a depth and a lateral size of the lesion.

FIG. 1

FIG. 2

EP 4 588 450 A1

*FIG. 3*

START *302* → INSERT CATHETER (PROBE) TIP INTO TISSUE AT POSITION TO BE ABLATED *304*

ESTIMATE INITIAL CATHETER TIP PENETRATION DEPTH USING: 1) IMPEDANCE, 2) CONTACT FORCE, AND 3) BLOOD CONDUCTIVITY (σ), MEASUREMENTS *306*

APPLY ABLATION CURRENT *308*

MEASURE TEMPERATURE AND IMPEDANCE USING SENSORS OF THE PROBE, AND CREATE A FINITE ELEMENT (FE) MODEL FROM THE MEASURE TEMPERATURE, IMPEDANCE, AND BLOOD CONDUCTIVITY TO DETERMINE LESION SIZE *310*

FE MODEL MATCHES MEASUREMENTS? *312* — NO → RE-ESTIMATE CATHETER TIP PENETRATION DEPTH *314*

YES

CREATE A REAL-TIME MODEL OF HOT SPOT TEMPERATURE AND PRESSURE VS. TIME FOR THE LESION SIZE *316*

PID CONTROLLER CHANGES THE RF GENERATOR CURRENT SUPPLY TO THE ABLATION CATHETER (TIP) *322*

TARGET (SETUP) TEMPERATURE *320*

HOT SPOT TEMPERATURE MEETS THE TARGET TEMPERATURE? *318* — NO

YES

IS ABLATION COMPLETE? *324* — NO

YES → END *326*

```
┌─────────────────────────────┐
│   OBTAIN BLOOD CONDUCTIVITY  │
│    (σ) FROM A BLOOD SAMPLE   │
│     FROM THE PATIENT 402     │
└─────────────────────────────┘
              │
              ▼
        ◇ IS σ < SET VALUE ◇          YES    ┌──────────────────────────┐
        (E.G., 4mS/cm) 404       ──────────▶  │        SELECT A          │
              │                               │  TEMPERATURE GUIDED      │
              │ NO                            │    CATHETER 406          │
              ▼                               └──────────────────────────┘
┌─────────────────────────────┐                         │
│      PROCEED WITH THE        │ ◀───────────────────────┘
│      PROCEDURE 408           │
└─────────────────────────────┘
              │
              ▼
        (  END 410  )
```

*FIG. 4*

## EUROPEAN SEARCH REPORT

Application Number

EP 25 15 0199

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/224573 A1 (BAR-TAL MEIR [IL] ET AL) 15 September 2011 (2011-09-15) * paragraphs [0086], [0089], [0091], [0092], [0117], [0118], [0136], [0140] - paragraphs [0142], [0144], [0147], [0153], [0165]; figures 3, 4, 6, 7 * | 8-15 | INV. A61B18/14 A61B18/00 |
| A | CN 115 300 094 A (SHANGHAI SHANGYANG MEDICAL TECH CO LTD) 8 November 2022 (2022-11-08) * the whole document * * paragraphs [0001], [0151], [0152]; figure 9 * | 1-15 | |
| A | US 2019/223944 A1 (COATES PAUL [US]) 25 July 2019 (2019-07-25) * paragraphs [0055] - [0057]; figure 6 * | 1-15 | |
| A | US 2014/235986 A1 (HARLEV DORON [US] ET AL) 21 August 2014 (2014-08-21) * paragraph [0139]; figure 5 * | 1-15 | TECHNICAL FIELDS SEARCHED (IPC) |
| A | CN 116 392 234 A (SHANGHAI HONGTONG IND CO LTD) 7 July 2023 (2023-07-07) * the whole document * * paragraph [0065]; figure 1 * | 1-15 | A61B |

The present search report has been drawn up for all claims

| Place of search | Date of completion of the search | Examiner |
|---|---|---|
| The Hague | 10 June 2025 | Bois, Nadège |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 0199

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2025

| Patent document cited in search report | Publication date | Patent family member(s) | | Publication date |
|---|---|---|---|---|
| US 2011224573 A1 | 15-09-2011 | NONE | | |
| CN 115300094 A | 08-11-2022 | NONE | | |
| US 2019223944 A1 | 25-07-2019 | CN | 111867507 A | 30-10-2020 |
| | | EP | 3742996 A1 | 02-12-2020 |
| | | JP | 7444784 B2 | 06-03-2024 |
| | | JP | 2021516594 A | 08-07-2021 |
| | | JP | 2024054381 A | 16-04-2024 |
| | | US | 2019223944 A1 | 25-07-2019 |
| | | US | 2023000549 A1 | 05-01-2023 |
| | | WO | 2019147470 A1 | 01-08-2019 |
| US 2014235986 A1 | 21-08-2014 | US | 2010286551 A1 | 11-11-2010 |
| | | US | 2012253161 A1 | 04-10-2012 |
| | | US | 2014235986 A1 | 21-08-2014 |
| | | US | 2017086705 A1 | 30-03-2017 |
| CN 116392234 A | 07-07-2023 | CN | 116392234 A | 07-07-2023 |
| | | CN | 120093410 A | 06-06-2025 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63617780
- US 55391199 B
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B
- US 10398348 B
- US 11596324 B
- US 9241756 B
- US 8900225 B

### Non-patent literature cited in the description

- **TEXTER, JR. et al.** The Electrical Conductivity of Blood - II. Relation and Cell Count. *Blood*, November 1950, vol. 5 (11), 1036-1048
- **J. KOSUTH et al.** Chamber-Specific Radiofrequency Lesion Dimension Estimation Using Novel Catheter-Based Tissue Interface Temperature Sensing. *JACC: Clinical Electrophysiology*, 2017, vol. 3 (10), 1092-1102
- **J. KOSUTH et al.** Chamber-Specific Radiofrequency Lesion Dimension Estimation Using Novel Catheter-Based Tissue Interface Temperature Sensing. *JACC: Clinical Electrophysiology*